# EUROPEAN PATENT APPLICATION

(11) **EP 2 348 026 A1**
(43) Date of publication of application: **27.07.2011**
(21) Application number: 09382291.4
(22) Date of filing: 22.12.2009
(51) Int. Cl.: C07D 513/04

(54) **Process for the preparation of brinzolamide and intermediates thereof**

(71) Applicant: Duke Chem, S. A., 08799 Olerdola Barcelona (ES)
(72) Inventor: Catasús Asenjo, Mònica, 08799, Olerdola (ES); Esteve Casol, Judit, 08799, Olerdola (ES); Gamero Valenzuela, Vanessa, 08799, Olerdola (ES)
(74) Representative: ZBM Patents

(57) **Abstract**

Preparation process of brinzolamide comprising the reaction of an halosubstituted precursor, wherein the ethylamine group is optionally protected, first with an halogen-metal exchange reaction, then with sulfur dioxide to form a sulfinate salt, and then reacting the sulfinate salt with an ammonia source. The process allows obtaining brinzolamide at an industrial scale with a high yield and high purity at reduced costs. Some new intermediates are used in this process.

## Description

The present invention relates to a process for the preparation of an active pharmaceutical ingredient known as brinzolamide, as well as to some new intermediates useful in such preparation process.

### BACKGROUND ART

Brinzolamide is the generic name of the (4R)-4-(ethylamino)-3,4-dihydro-2-(3-methoxypropyl)-2H-thieno[3,2-e]-1,2-thiazine-6-sulfonamide 1,1-dioxide, having the structural formula (I).

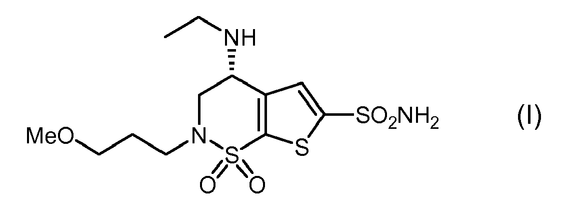

Brinzolamide is an inhibitor of carbonic anhydrase. This enzyme, found in many tissues of the body including the eye, catalyzes the reversible reaction involving the hydration of carbon dioxide and the dehydration of carbonic acid. Inhibition of carbonic anhydrase in the ciliary processes of the eye decreases aqueous humor secretion, presumably by slowing the formation of bicarbonate ions with subsequent reduction in sodium and fluid transport. The result is a reduction in intraocular pressure (IOP). Elevated intraocular pressure is a major risk factor in the pathogenesis of optic nerve damage and glaucomatous visual field loss.

In EP-A-527801, (R)-3,4-dihydro-4-alkylamino-2-substituted-2H-thieno[3,2-e]-1,2-thiazine-6-sulfonamide-1,1-dioxides, including brinzolamide, are prepared by resolution of the racemate via the di-p-toluyl-D-tartaric acid salt: 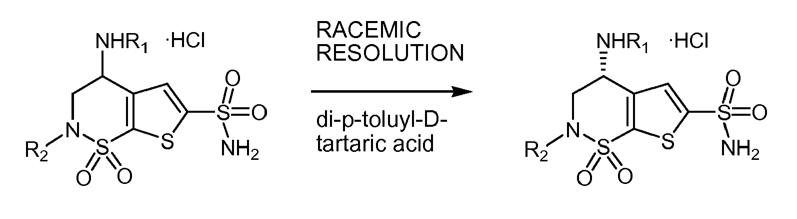 where R₁ is alkyl, and R₂ can be different radicals. In this case, the resolution of the racemic compound is carried out at the last synthetic step, what implies a considerable increase of costs.

The same document discloses a process for the preparation of the mentioned compounds from (S)-3,4-dihydro-4-hydroxy-2-substituted-2H-thieno[3,2-e]-1,2-thiazine-6-sulfonamide-1,1-dioxides by activation of the C(4)-hydroxyl group and, subsequently, displacement with the appropriate amine:

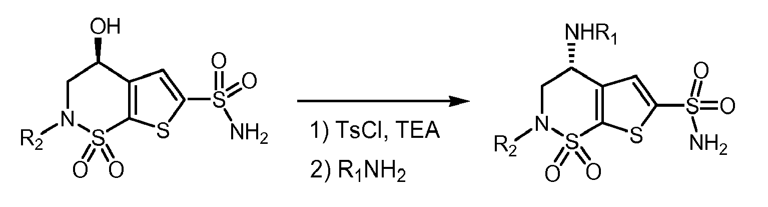

In this process, the introduction of the amine substituent at the end of the synthetic route leads to the final product with relatively low yields. Thus, for instance, in the case of brinzolamide the corresponding hydrochloride is obtained with only a 34% of yield.

In EP-A-617038 a process for the preparation of (R)-3,4-dihydro-4-alkylamino-2-substituted-2H-thieno[3,2-e]-1,2-thiazine-6-sulfonamide-1,1-dioxides from 3-acetyl-2,5-dichlorothiophene is disclosed. This process involves protection of the sulfonamide group in the last stage and requires the use of very low temperatures and highly inflammable solvents, what makes it difficult to scale-up to an industrial level. According to this method, overall yield of brinzolamide through Example 3 was less than 48% (61%x78%).

WO2008/62463 discloses a process for the preparation of brinzolamide wherein the introduction of the alkylamine substituent is carried out at an earlier stage and before sulfonamidation reaction. This process involves the use of a chromatographic column in order to obtain the final product in a very low yield. Particularly, overall yield of brinzolamide through Example 2 was less than 20% (71%x28%), what makes it unsuitable as an industrial process.

WO2008/132114 discloses a process for the preparation of brinzolamide wherein the introduction of the alkylamine substituent is carried out at an earlier stage and before sulfonamidation reaction, which is performed over and intermediate compound with a protected alkylamine. According to this method, overall yield of brinzolamide through Example 5, 6 and 7 was of about 47% (72%x80%x86%x95%).

Despite the teaching of these prior art documents, the provision of an improved process for the preparation of brinzolamide in higher yields and milder conditions is still a matter of great interest in industry.

### SUMMARY OF THE INVENTION

Inventors have found a new process for the preparation of brinzolamide that overcomes or minimizes the drawbacks of the processes disclosed in the prior art. The new process allows obtaining brinzolamide with unexpectedly high overall yields and high purity. The process is easy to scale-up to an industrial level, and is more cost-effective than the already known processes.

Accordingly, a first aspect of the present invention refers to a process for the preparation of brinzolamide of formula (I), or a pharmaceutically acceptable salt thereof, 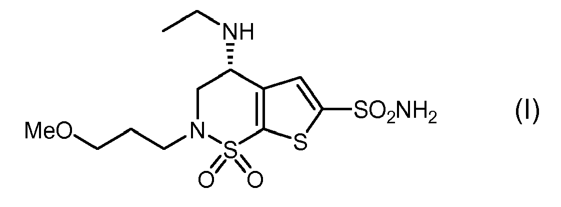 comprising a) reacting a compound of formula (III) 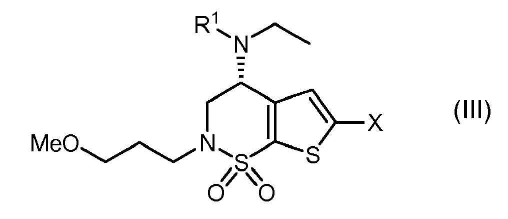 wherein R¹ is H or a protecting group (PG), and X is an halogen group selected from CI, Br, and I, with an alkyl lithium or alkyl magnesium halide; then reacting the resulting anion with sulfur dioxide to form a sulfinate salt; and then reacting the sulfinate salt directly with an aminating reagent, such as hydroxylamine-*O*-sulfonic acid, or the sulfinate salt can be first chlorinated with a chlorinating reagent, such as N-chlorosuccinimide, to its corresponding sulfonyl chloride wich in turn can be aminated with ammonia to give, when R¹ is H, directly the final compound of formula (I), or, when R¹ is PG, a compound of formula (II) 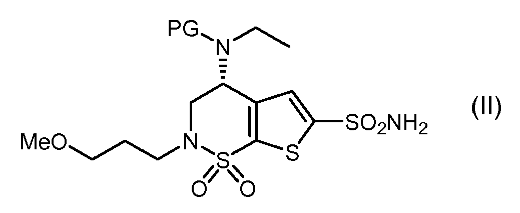 wherein PG is a protecting group; b) when R¹ is PG, submitting the compound of formula (II) to a deprotection reaction to yield the compound of formula (I); and c) optionally, treating the compound of formula (I) obtained with a pharmaceutically acceptable acid to form the corresponding pharmaceutically acceptable salt.

This process differs from the above mentioned prior art processes in that the sulfonamidation reaction on C(6) is carried out over a C(4) ethylamine intermediate wherein position C(6) is halosubstituted (i.e. compound of formula (III)). Surprisingly, inventors have found that the process of the invention allows to obtain brinzolamide with unexpectedly higher overall yield than the ones obtained by the processes known in the prior art, even without the need to protect the alkylamine on C(4), and at the same time with a high purity. This results in the global process being more cost-effective. In particular, when the sulfonamidation is carried out after protecting the ethylamino moiety, the process is especially advantageous since much higher yield and purity are obtained.

According to a second aspect of the invention, new compounds of formula (III') and (III) 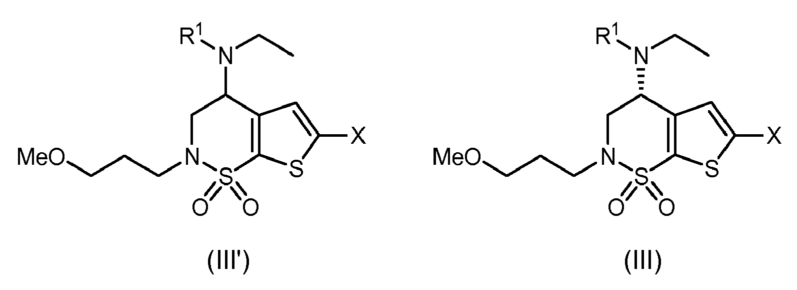 wherein R¹ is H or a protecting group, and X is an halogen group selected from Cl, Br, and I,or a salt thereof, are provided as intermediates useful for the preparation of brinzolamide.

The compound of formula (V) 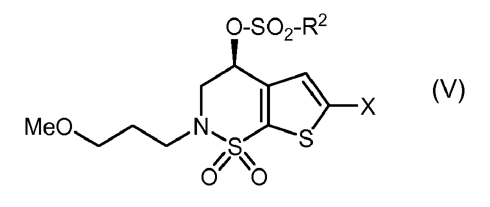 wherein X is an halogen group selected from Cl, Br, and I, and R² is a radical selected from the group consisting of (C₁-C₄)-alkyl, CF₃, phenyl, and phenyl mono- or disubstituted by a radical selected from the group consisting of (C₁-C₄)-alkyl, bromo, and nitro, which is a further intermediate useful for the preparation of brinzolamide, also form part of the present invention.

These compounds allow the preparation of brinzolamide or salts thereof by a process which proceeds with high yields and purity. Therefore, it is also part of the invention the use of the compounds of formula (III) or (III') or of the compound of formula (V) as intermediates for the preparation of brinzolamide or pharmaceutically acceptable salts thereof.

### DETAILED DESCRIPTION OF THE INVENTION

As mentioned above, brinzolamide, or its pharmaceutically acceptable salts, can be prepared by a process comprising submitting a compound of formula (III) 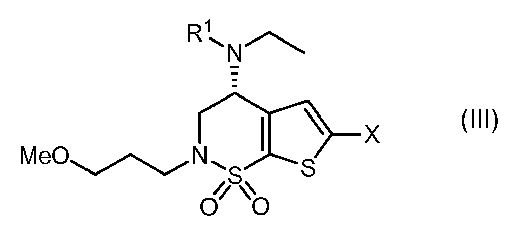 wherein R¹ is H or a protecting group (PG), and X is an halogen group selected from Cl, Br, and I, to a sulfonamidation reaction; and when R¹ is PG, submitting the obtained compound to a deprotection reaction; and, optionally, treating brinzolamide with a pharmaceutically acceptable acid to form the corresponding pharmaceutically acceptable salt.

The compound of formula (III) can be in the form of free base or of a salt. In the case that the starting material (III) is in the form of a salt, the reaction is carried out in the presence of a base in order to convert the salt into the free base.

Brinzolamide can be isolated directly from the reaction medium as a base or as a salt.

The pharmaceutically acceptable salts of the compound of formula (I) include acid addition salts such as the hydrochloride, hydrobromide, hydrofluoride, hydroiodide, sulphate or bisulphate, phosphate, hydrogen phosphate, acetate, besylate, citrate, fumarate, gluconate, lactacte, maleate, mesylate, succionate or tartrate salts. Preferably, the salt of the compound of formula (I) is the hydrochloride salt.

These salts can be prepared conventionally, for instance, by mixing a solution of the free base with the corresponding acid in a suitable solvent system, e.g. ethanol or tetrahydrofuran, and recovering the acid addition salt either as precipitate or by evaporation of the solvent from the solution. The solvent system can be either aqueous, non-aqueous or a partially aqueous medium. Usually, the salts are prepared by contacting stoichiometric amounts of the acid and basic entities. Brinzolamide or its salts can exist in solvated, as well as unsolvated forms, including hydrated forms, i.e. the brinzolamide can contain in its structure stoichiometric amounts of solvent in the case of solvates, or of water in the case of hydrates. It is to be understood that the invention encompasses all such solvated, as well as unsolvated forms. Obtaining a solvate or a hydrate depends on the solvent used and the crystallization conditions, which can be determined by the skilled person.

Scheme I below illustrates a particular embodiment of the general process of the invention:

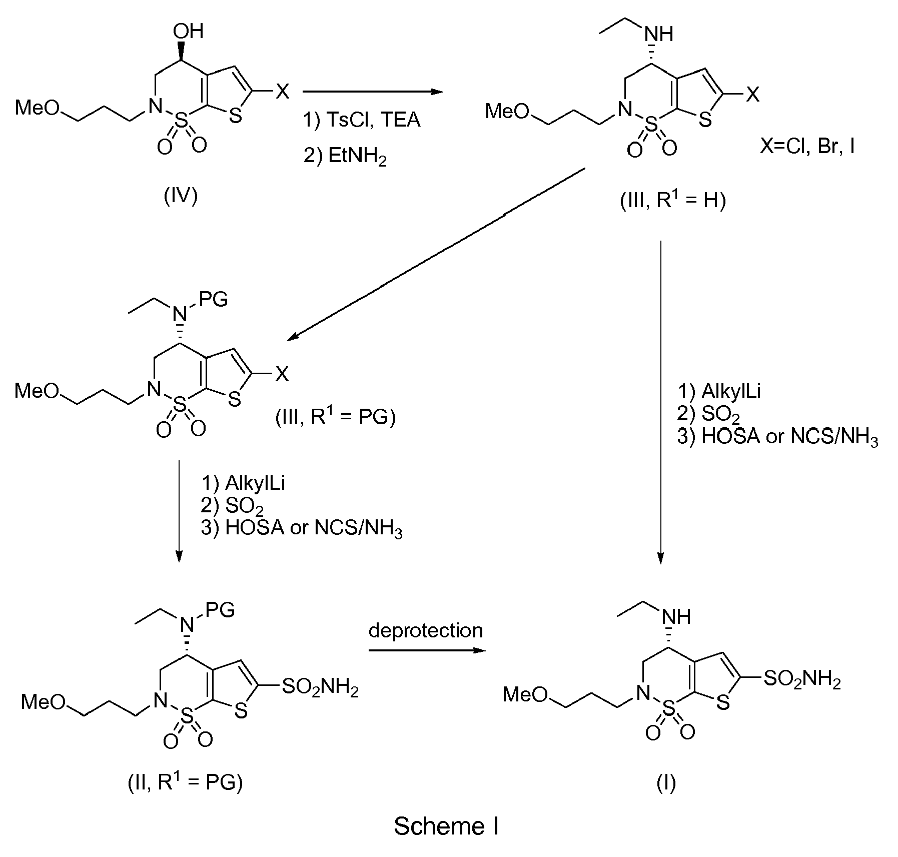

Thus, compound of formula (I), i.e. brinzolamide, can be obtained by two methods. According to method A, brinzolamide is obtained directly from sulfonamidation of compound (III) wherein R¹ is H. According to method B, the alkylamine moiety of compound (III) wherein R¹ is H is protected previously to carry out the sulfonamidation reaction, and finaly deprotection is carried out to obtain brinzolamide.

In both methods A and B, formation of sulfonamide group at C(6) can be accomplished in three stages. First, a C(6) anion is formed by halogen-metal exchange in a polar aprotic solvent. Preferably, from 2 to 5 equivalents of an alkyl lithium or alkyl magnesium halide are used. More preferably, from 2.5 to 4 equivalents. Also preferably, the alkyl lithium is n-butyl lithium. The reaction is carried out at a temperature of -70 to 0°C. More preferably, the reaction is carried out at -30 to 0°C. In the second stage, the anion thus formed is reacted with sulfur dioxide to form an intermediate sulfinate salt. This is accomplished by passing sulfur dioxide gas into the solution of the anion at the same temperature until the pH of the solution is acidic. In the third stage, the solvent is removed, the solid sulfinate salt is dissolved in water and treated with hydroxylamine-O-sulfonic acid (HOSA) in the presence of sodium acetate trihydrate at a temperature of -5 to 30°C to yield the corresponding 6-sulfonamide. This last stage can also be carried out by reaction of the sulfinate salt with a source of positive halogen, such as N-chlorosuccinimide (NCS), followed by treatment with NH₃.

The polar aprotic solvent used in the present invention can be selected from ketones, such as acetone or ethyl methyl ketone, nitriles such as acetonitrile, (C₃-C₁₀)alkyl ethers such as diethyl ether, diisopropyl ether, and methyl tert-butyl ether, cicloalkyl ethers such as tetrahydrofuran and dioxane, dimethylformamide, dimethylacetamide, dimethylsulfoxide, N,Ndimethylpyrrolidinone, sulfolane or mixture thereof. Preferably the polar aprotic solvent is tetrahydrofuran.

Method A allows to obtain a brinzolamide with a higher overall yield and a higher purity than the ones obtained by the methods of the prior art. After carrying out two additional purification steps, brinzolamide fulfilling the requirements of the USP is obtained with an overall yield of around 52%. As a comparison, the additional purifications required by the product obtained by the method disclosed in EP-A-617038 in order to fulfill pharmacopoeia requirements provided an overall yield lower to 44%.

According to method B, the compound of formula (III) wherein R¹ is a protecting group (PG), can be obtained by a protection reaction of the secondary amine of a compound of formula (III) wherein R¹ is H. Examples of protecting groups for amino are given in T. W. Greene and P. G. M. Wuts, Protective Groups in Organic Synthesis, (Wiley, 3rd ed. 1999), pp. 495-653. Protecting groups can be easily removed when the desired reaction is complete. Among the representative protecting groups for amino those in which the amino group is acylated, such as in the form of an N-alkyl or an N-arylamide, or converted to a carbamate are included.

When carrying out method B, namely when the halogen-metal exchange is carried out on the intermediate with the protected amine, even higher overall yield and purity are obtained. Additionally, purification in order to obtain a final product of the desired purity is easier to carry out. Only one purification step is needed in order to fulfill pharmacopoeia requirements. This is evidenced by the examples below where an overall yield of around 61 % is obtained, which is significantly higher than the one obtained after the several purifications required by the product obtained by the method disclosed in EP-A-617038 in order to fulfill pharmacopoeia requirements. This higher yield and purity are achieved even if the reaction is carried out at higher temperatures. Thus, unlike most of the prior art processes, method B allows to carry out the halogen-metal exchange reaction at -30 to 0°C, instead of the -60°C or below used in the prior art, which is advantageous since the process is easier to carry out at industrial scale. Accordingly, in a preferred embodiment of the first aspect of the invention R¹ is a protecting group, namely the sulfonamidation reaction is carried out over the corresponding intermediate with the alkylamine moiety protected. In a more preferred embodiment, the protecting group is an alkyl or acyl group.

The compound of formula (III) wherein R¹ is H can be obtained through the resolution of the corresponding racemate. Accordingly, in a preferred embodiment the process of the invention further comprises submitting the corresponding racemate of formula (III') wherein R¹ is H 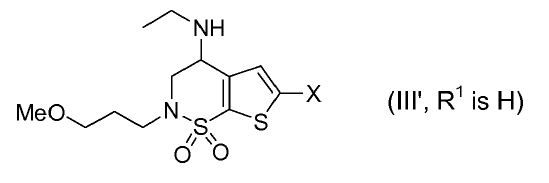 and wherein X is an halogen group selected from Cl, Br, and I, to a resolution process to yield the compound of formula (III) wherein R¹ is H and X is as defined above. Preferably, the resolution will be carried out with an optically active acid. Generally weak optically active carboxylic acids are preferred. Suitable optically active carboxylic acid resolving agents include tartaric acid, diacetyl tartaric acid, dibenzoyl tartaric acid, di-p-toluyl tartaric acid, malic acid, camphoric acid, menthoxyacetic or mandelic acid. More preferably the optically active acid is (-)-di-p-toluyl-tartaric acid. The solvent(s) will have to be properly chosen to effectuate the precipitation of the diastereomeric salt of the compound of formula (III) wherein R¹ is H as is well known to those skilled in the art.

Preferably, the compound of formula (III) wherein R¹ is H is prepared from the compound of formula (IV) 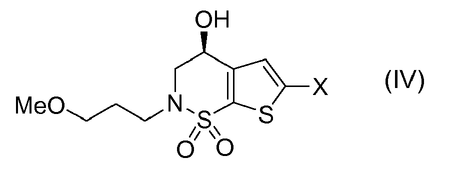 wherein X is as defined above, by activation of the hydroxyl group and displacement of the activated hydroxyl group with ethylamine, with inversion of the stereochemistry at C(4). So, according to a preferred embodiment, the process of the invention further comprises submitting the hydroxyl group of a compound of formula (IV) as defined above to an activation reaction, and subsequently displacing the activated hydroxyl group with ethylamine.

The activation of the hydroxyl group can be performed by reaction with a sulfonation agent in the presence of a base in a suitable solvent system, preferably in a polar aprotic solvent such as tetrahydrofurane. So, in a preferred embodiment the alcohol of formula (IV) is reacted with a sulfonation agent selected from methanesulfonic anhydride and a sulfonyl chloride of formula Cl-SO₂-R², wherein R² is a radical selected from (C₁-C₄)-alkyl, CF₃, phenyl, and phenyl mono- or disubstituted by a radical selected from (C₁-C₄)-alkyl, bromo and nitro, in the presence of an base, to give the compound of formula (V) 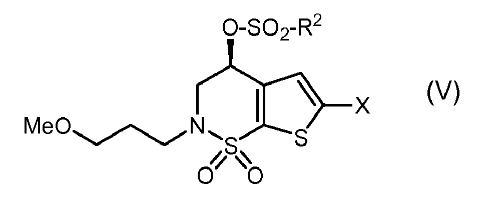 wherein R² and X are as defined above. Preferably, R² is p-tolyl.

The sulfonation agent includes methanesulfonyl chloride, p-toluenesulfonyl chloride, benzenesulfonyl chloride, p-bromotoluenesulfonyl chloride, p-nitrobenzenesulfonyl chloride, and trifluoromethanesulfonyl chloride. The activation base includes triethylamine, pyridine, 4-dimethylaminopyridine, and N-methylmorpholine.

To perform the activation of the hydroxyl group, p-toluenesulfonyl chloride and triethylamine (TEA) are preferred. Use of 1.5 to 2.5 equivalents of p-toluenesulfonyl chloride and triethylamine at a temperature of -10 to 30°C for a period of 2 to 20 hours are preferred. After tosylation is completed, the displacement reaction is accomplished by adding the ethylamine to the cold solution. Preferably, the reaction is carried out by adding 4 to 40 equivalents of ethylamine at a temperature of -10 to 30°C, more preferably at 0°C. After a period of 4 to 40 hours at a temperature of 15 to 50°C, more preferably at ambient temperature, the product is isolated by an acid-base work-up.

The process of the present invention includes single reaction steps of the global process to obtain brinzolamide, as well as the combination of two of more sequential steps of the global process described above, the process being able to be carried out in sequential steps isolating the intermediates obtained, or alternatively, in one pot without isolation of any intermediate compound.

The racemic compound of formula (III') can be prepared by following the process represented in Scheme I, but starting with the corresponding racemic alcohol of formula IV'.

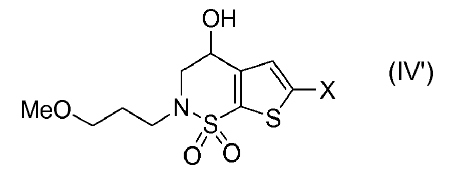

As mentioned above the compound of formula (III) and (III'), and compound of formula (V) are new and form part of the invention. It also forms part of the invention the use of these compounds as intermediates for the preparation of brinzolamide or its salts.

The preparation of the compound of formula (IV) can be accomplished by bromination of the compound of formula (VI), for instance with pyridinium bromide perbromide (PBP), and subsequent reduction of the obtained compound of formula (VIII) with (+)-β-chlorodiisopinocamphenyl-borane, followed by treatment with aqueous base, as shown in Scheme II:

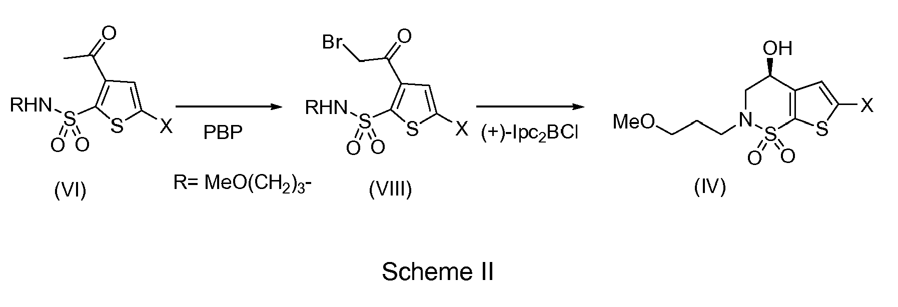

The racemic alcohol of formula (IV') can be obtained as shown in Scheme 11 by conversion of acyclic sulfonamide (VI) but by using NaBH₄ instead of (+)-Ipc₂BCl to reduce the compound of formula (VIII).

Thus, taking into account all the advantages above mentioned, clearly the alternative process to obtain brinzolamide of the present invention can be considerably more efficient and advantageous than those previously disclosed in the art.

Additional objects, advantages and features of the invention will become apparent to those skilled in the art upon examination of the description or may be learned by practice of the invention. The following examples and drawings are provided by way of illustration, and are not intended to be limiting of the present invention.

### EXAMPLES

### Example 1. Preparation of (R)-3,4-dihydro-4-ethylamino-6-chloro-2-(3-methoxypropyl)-2H-thieno[3,2-e]-1,2-thiazine-1,1-dioxide hydrochloride (III, R¹=H,X=Cl)

To a solution of -(S)-3,4-dihydro-6-chloro-4-hydroxy-2-(3-methoxypropyl)-2H thieno[3,2-e]-1,2-thiazine-1,1 -dioxide (100 g, 0.321 mol) and triethylamine (102.5 mL, 0.735 mol) in tetrahydrofurane (500 mL), cooled to 0°C, tosyl chloride (128.4 g, 0.674 mol) was added. Once the addition was finished, the reaction mixture was allowed to warm to room temperature and stirred for two hours more.

To the resulting suspension, cooled to 0°C, an aqueous solution of ethylamine 70% (516 mL, 0.641 mol) was added. Afterwards, the mixture was allowed to warm to room temperature overnight.

To the reaction mixture water (50 mL) and ethyl acetate (100 mL) were added. The two phases were separated and the organic layer was acidificated with concentrated hydrochloric acid to pH=1-3. The corresponding hydrochloric salt was precipitated and it was collected by filtration
to provide 98.7 g (82% yield) of (R)-4-(N-ethylamino)-3,4-dihydro-6-chloro-2-(3-methoxypropyl)-2H-thieno[3,2-e]-1,2-thiazine-1,1 -dioxide hydrochloride as a white crystalline solid.

### Example 2. Preparation of (R)-3,4-dihydro-4-ethylamino-2-(3-methoxypropyl)-2H-thieno[3,2-e]-1,2-thiazine-6-sulfonamide 1,1-dioxide (brinzolamide)

The (R)-4-(N-ethylamino)-3,4-dihydro-6-chloro-2-(3-methoxypropyl)-2H-thieno[3,2-e]-1,2-thiazine-1,1-dioxide hydrochloride (50 g, 0.133 mol) was treated with a solution of sodium bicarbonate and extracted with ethyl acetate to isolate the amine base form.

A solution of this crude (0.133 mol) in tetrahydrofurane (900 mL) was cooled to -40°C and n-butyl lithium 2.5 M in hexane (213 mL, 0.533 mol) was added drop wise. The reaction mixture was stirred at the same temperature for 1 h. Afterwards, anhydrous sulfur dioxide was bubbled through the reaction mixture until it was acidic. The resulting suspension was allowed to warm to room temperature. Then, water (450 mL) was added, and tetrahydrofurane was concentrated in vaccuo. To this solution sodium acetate trihydrate (108.6 g, 0.798 mol) and hydroxylamine-O-sulfonic acid (60.2 g, 0.532 mol) were added at 0°C. The resulting mixture was allowed to warm to room temperature and stirred overnight.

To the reaction mixture ethyl acetate (450 mL) was added. Before two phases were separated the mixture was acidificated with concentrated hydrochloric acid to pH=1-3. Then two phases were separated. The organic layer was extracted twice with hydrochloric acid 4N solution, and the combined aqueous layer was basified with sodium bicarbonate till pH=7.5-8. This suspension was stirred for 2 more hours while a solid was precipitated. The solid was collected by filtration and recrystallized twice from 2-propanol to provide 32.1 g (63% yield) of (R)-3,4-dihydro-4-ethylamino-2-(3-methoxypropyl)-2H-thieno[3,2-e]-1,2-thiazine-6-sulfonamide 1,1-dioxide as a white crystalline solid USP grade.

### Example 3. Preparation of (R)-4-(N-acetyl-N-ethylamino)-3,4-dihydro-6-chloro-2-(3-methoxypropyl)-2H-thieno[3,2-e]-1,2-thiazine-1,1-dioxide (III, X = Cl, R¹ = -CO-CH₃)

(R)-4-(N-ethylamino)-3,4-dihydro-6-chloro-2-(3-methoxypropyl)-2H-thieno[3,2-e]-1,2-thiazine-1,1-dioxide hydrochloride (40 g, 0.107 mol) was treated with an aqueous solution of sodium bicarbonate and extracted with ethyl acetate to isolate the amine base form.

To a solution of this crude (0.107 mol), and triethylamine (19 mL, 0.137 mol) in ethyl acetate (635 mL), cooled to 0°C, acetyl chloride (9.5 mL, 0.134 mol) was added dropwise. The reaction mixture was stirred at room temperature until HPLC showed the absence of starting material.

To the reaction mixture, water (635 mL) was added and two phases were separated. The organic layer was extracted twice with a saturated solution of bicarbonate and the ethyl acetate solution was concentrated in vaccuo. To this crude, heptane (200 mL) was added, and the white solid precipitated was crystallized immediately to give 37.5 g (92% yield) of (R)-4-(N-acetyl-N-ethylamino)-3,4-dihydro-6-chloro-2-(3-methoxypropyl)-2H-thieno[3,2-e]-1,2-thiazine-1,1-dioxide as a white crystalline solid.

### Example 4. Preparation of (R)-4-(N-acetyl-N-ethylamino)-3,4-dihydro-2-(3-methoxypropyl)-2H-thieno[3,2-e]-1,2-thiazine-6-sulfonamide-1,1-dioxide (II, R¹ = -CO-CH₃)

A solution of (R)-4-(N-acetyl-N-ethylamino)-3,4-dihydro-6-chloro-2-(3-methoxypropyl)-2H-thieno[3,2-e]-1,2-thiazine-1,1-dioxide (30 g, 0.079 mol) in tetrahydrofurane (600 mL) was cooled to -20°C and n-butyl lithium 2.5 M in hexane (96 mL, 0.240 mol) was added dropwise. The reaction mixture was stirred at the same temperature for 1 h. Afterwards, anhydrous sulfur dioxide was bubbled through the reaction mixture until it was acidic. The resulting suspension was allowed to warm to room temperature water (300 mL) was added and tetrahydrofurane was evaporated in vaccuo. To this solution sodium acetate trihydrate (64.5 g, 0.474 mol) and hydroxylamine-O-sulfonic acid (35.7 g, 0.316 mol) were added at 0°C. The resulting mixture was allowed to warm to room temperature and stirred overnight.

To the reaction mixture ethyl acetate (300 mL) was added and two phases were separated. The aqueous layer was extracted twice with ethyl acetate. The combined organic layers were washed with a saturated solution of sodium bicarbonate, and then the ethyl acetate solution was concentrated in vaccuo to get 28.6 g (85% yield) of (R)-4-(N-acetyl-N-ethylamino)-3,4-dihydro-2-(3-methoxypropyl)-2H-thieno[3,2-e]-1,2-thiazine-6-sulfonamide-1,1-dioxide as a semi-solid product, which one was used without further purification in the next step of the synthesis.

### Example 5. Preparation of (R)-3,4-Dihydro-4-ethylamino-2-(3-methoxypropyl)-2H-thieno[3,2-e]-1,2-thiazine-6-sulfonamide 1,1-dioxide (brinzolamide)

To the (R)-4-(N-acetyl-N-ethylamine)-3,4-dihydro-2-(3-methoxypropyl)-2H-thieno[3,2-e]-1,2-thiazine-6-sulfonamide-1,1-dioxide (25 g, 0.059 mol) water (935 mL) and sulphuric acid (206 mL) were added. The resulting mixture was heated at reflux until HPLC showed the absence of starting material.

The crude reaction was cooled to room temperature and it was basificated with NaOH 50% to pH=7.5-8. To this suspension ethyl acetate (625 mL) was added and two phases were separated. The water layer was extracted twice with ethyl acetate and the combined organic layers were concentrated in vaccuo and crystallized once from 2-propanol to provide 21.5 g (95% yield) of (R)-3,4-dihydro-4-ethylamino-2-(3-methoxypropyl)-2H-thieno[3,2-e]-1,2-thiazine-6-sulfonamid-1,1-dioxide as a white crystalline solid USP grade.

## Claims

1. A process for the preparation of brinzolamide of formula (I), or a pharmaceutically acceptable salt thereof, 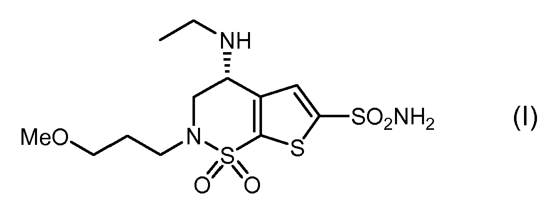 comprising:
a) reacting a compound of formula (III) 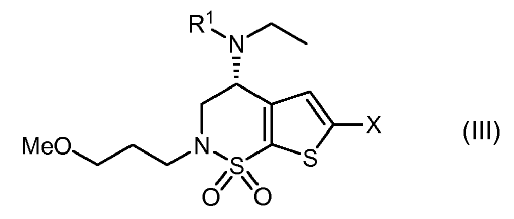 wherein R¹ is H or a protecting group (PG), and X is an halogen selected from Cl, Br, and I, with an alkyl lithium or alkyl magnesium halide; then reacting the resulting anion with sulfur dioxide to form a sulfinate salt; and then reacting the sulfinate salt directly with an aminating reagent, such as hydroxylamine-O-sulfonic acid, or the sulfinate salt can be first chlorinated with a chlorinating reagent, such as N-chlorosuccinimide, to its corresponding sulfonyl chloride wich in turn can be aminated with ammonia to give, when R¹ is H, directly the final compound of formula (I), or, when R¹ is PG, a compound of formula (II) 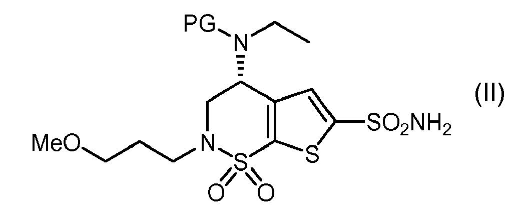 wherein PG is a protecting group;
b) when R¹ is PG, submitting the compound of formula (II) to a deprotection reaction to yield the compound of formula (I), and
c) optionally, treating the compound of formula (I) obtained with a pharmaceutically acceptable acid to form the corresponding pharmaceutically acceptable salt.

2. The process according to claim 1, wherein R¹ is a protecting group.

3. The process according to any one of claims claims 1 or 2, wherein the protecting group is an alkyl or acyl group.

4. The process according to any one of claims 1 to 3, further comprising submitting the corresponding racemate of formula (III') wherein R¹ is H 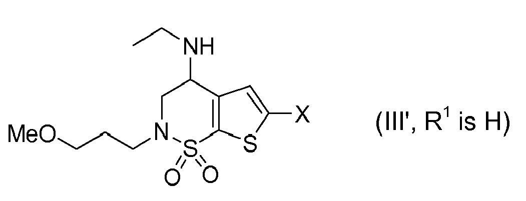 and wherein X is an halogen group selected from Cl, Br, and I, to a resolution process to yield the compound of formula (III) wherein R¹ is H and X are as defined above.

5. The process according to claim 4, wherein the resolution is carried out with an optically active carboxylic acid

6. The process according to any one of claims 1 to 3, further comprising submitting the hydroxyl group of a compound of formula (IV) 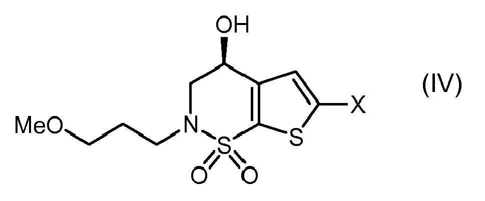 wherein X is an halogen group selected from Cl, Br, and I,
to an activation reaction, and subsequently displacing the activated hydroxyl group with ethylamine.

7. The process according to claim 6, wherein the activation reaction is carried out by reacting the alcohol of formula (IV) with a sulfonating agent selected from methanesulfonic anhydride and a sulfonyl chloride of formula Cl-SO₂-R², wherein R² is a radical selected from the group consisting of (C₁-C₄)-alkyl, CF₃, phenyl, and phenyl mono- or disubstituted by a radical selected from the group consisting of (C₁-C₄)-alkyl, bromo and nitro, in the presence of a base, to give a compound of formula (V) 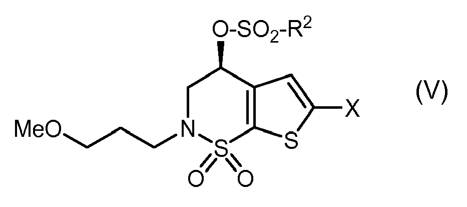 wherein X is an halogen group selected from Cl, Br, and I, and R² is as defined above.

8. The process according to claim 7, wherein R² is p-tolyl.

9. A racemic compound of formula (III') or its (R)-stereoisomer of formula (III) 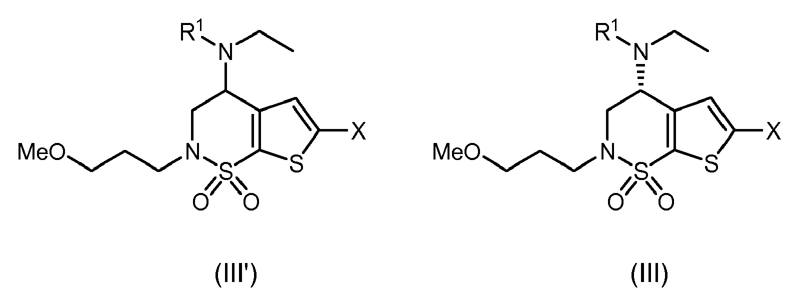 wherein R¹ is H or a protecting group, and X is an halogen group selected from Cl, Br, and I, or a salt thereof.

10. A compound of formula (V) 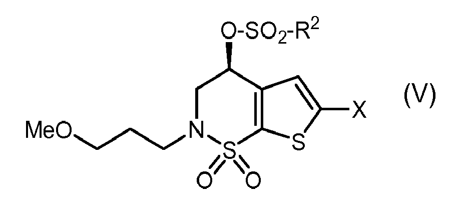 wherein X is an halogen group selected from Cl, Br, and I, and R² is a radical selected from the group consisting of (C₁-C₄)-alkyl, CF₃, phenyl, and phenyl mono- or disubstituted by a radical selected from the group consisting of (C₁-C₄)-alkyl, bromo, and nitro.
